# EUROPÄISCHE PATENTSCHRIFT

(11) **EP 0 659 733 B1**
(45) Veröffentlichungstag und Bekanntmachung des Hinweises auf die Patenterteilung: **13.08.1997**
(21) Anmeldenummer: 94118077.0
(22) Anmeldetag: 17.11.1994
(51) Int. Cl.: C07C 209/48, C07C 209/26

(54) **Verfahren zur Beeinflussung des cis-/trans-Isomerenverhältnisses von 3-Aminomethyl-3,5,5-Trimethylcyclohexylamin bei dessen Herstellung aus 3-Cyano-3,5,5-trimethylcyclohexanon**
Process for influencing the cis-/trans-relation of 3-aminomethyl-3,5,5-trimethylcyclohexylamine in its preparation from 3-cyano-3,5,5-trimethylcyclohexanone
Procédé pour influencer la proportion cis-/trans- de la 3-aminométhyl-3,5,5-triméthylcyclohexylamine dans la préparation à partir de 3-cyano-3,5,5-triméthylcyclohexanone

(30) Priorität: 22.12.1993 DE 4343891
(43) Veröffentlichungstag der Anmeldung: 28.06.1995
(73) Patentinhaber: Degussa Aktiengesellschaft, 60311 Frankfurt (DE)
(72) Erfinder: Haas, Thomas Dr., D-60316 Frankfurt (DE); Arntz, Dietrich Dr., D-61440 Oberursel (DE); Most, Dieter Dr., D-63486 Bruchköbel (DE)

(56) Entgegenhaltungen:
- EP-A- 0 503 246
- EP-A- 0 534 449

## Beschreibung

Die Erfindung richtet sich auf ein Verfahren zur Beeinflussung des cis/-trans-Isomerenverhältnisses von 3-Aminomethyl-3,5,5-trimethylcyclohexylamin (Isophorondiamin; IPDA) bei dessen Herstellung aus 3-Cyano-3,5,5-trimethylcyclohexanon (Isophornnitril; IPN). Die Herstellung des Isophorondiamins basiert auf der aminierenden Hydrierung von Isophoronnitril mit Ammoniak und Wasserstoff in Gegenwart eines Hydrierkatalysators.

Isophorondiamin wird als Ausgangsprodukt zur Herstellung von Isophorondiisocyanat, einer Isocyanatkomponente für Polyurethansysteme, als Aminkomponente für Polyamide und als Härter für Epoxidharze verwendet. Isophorondiamin wird üblicherweise aus Isophoronnitril hergestellt, wobei in Gegenwart von Ammoniak, Wasserstoff und üblichen Hydrierkatalysatoren die Carbonylgruppe in eine Aminogruppe und die Nitrilgruppe in eine Aminomethylgruppe überführt werden. Das Ausgangsprodukt Isophoronnitril läßt sich in bekannter Weise durch Anlagerung von Cyanwasserstoff an Isophoron erhalten - siehe DE-OS 39 42 371.

Isophorondiamin kann in unterschiedlichen Isomeren vorkommen, gemäß Die Angewandte Makromolekulare Chemie, 153 (1987) 1-13 (Nr. 2502) wurde für das in handelsüblichem Isophorondiamin zu etwa 75 % vorliegende Isomere eine Sesselkonformation mit einer cis-Anordnung der equatorialen Aminogruppe am C¹-Atom und der equatorialen Aminomethylgruppe am C³-Atom festgestellt; bei dem zu etwa 25 % anwesenden Isomeren handelt es sich um das trans-Isomere mit equatorialer Amino- und axialer Methylaminogruppe.

Das cis- und trans-Isomere von Isophorondiamin sowie das hieraus zugängliche cis- und trans-Isomere von Isophorondiisocyanat weisen unterschiedliche Reaktivitäten auf, was für die vorgesehene Anwendung von technischer Bedeutung sein kann. So lehrt die DE-OS 42 11 454, daß durch Verwendung eines Isophorondiamin-Isomerengemischs, bestehend aus über 40 % des trans-Isomeren und unter 60 % des cis-Isomeren als Reaktionskomponente in Polyadditionsharzen, wie insbesondere Epoxidharzen, sowohl die Topfzeit verlängert als auch die maximale Härtungstemperatur erniedrigt werden. Zur Erzielung einer möglichst hohen Reaktionsgeschwindigkeit werden umgekehrt Isophorondiamin-Isomerengemische bevorzugt, welche einen möglichst hohen Anteil an dem cis-Isomeren aufweisen.

Die Herstellung von Isophorondiamin aus Isophoronnitril wird zwar in vielen Dokumenten beschrieben, jedoch wird in diesen keine Aussage über die Zusammensetzung des Isomerengemischs gemacht. Während handelsübliche Produkte ein cis-/trans-Isomerenverhältnis um 75 zu 25 aufweisen, wurden aus der DE-OS 42 11 454 auch IPDA-Isomerengemische bekannt, welche zwischen 50 und 70 % des trans-Isomeren enthalten.

Bezüglich bekannter Verfahren zur Herstellung von Isophorondiamin durch aminierende Hydrierung von Isophoronnitril wird beispielhaft auf die US 3,352,913 , die EP-B 0 042 119, die EP-A 0 449 089, die EP-A 0 394 967 und die JP-A 4-300852 verwiesen. In keinem dieser Dokumente werden Angaben zum Isomerenverhältnis gemacht.

Gemäß dem in der US 3,352,913 beschriebenen Verfahren wird Isophoronnitril mit Ammoniak in Gegenwart von an sich bekannten kobalt-, nickel-, eisen- oder edelmetallhaltigen Katalysatoren bei 50 bis 150 °C und einem Druck von wenigstens 50 bar, beispielsgemäß 120 bis 150 bar, aminierend hydriert. Die Hydrierung kann in An- oder Abwesenheit von organischen Lösungsmitteln erfolgen; Methanol wird bevorzugt. Der Hydrierkatalysator kann in Form von Suspensions- oder Festbettkatalysatoren eingesetzt werden.

Eine zweistufige Umsetzung, wobei in der ersten Stufe Isophoronnitril in 3-Cyano-3,5,5-trimethyl-iminocyclohexan überführt und dieses in der zweiten Stufe zu IPDA hydriert wird, lehrt die DE-OS 30 11 656. Auch die EP-B 0 042 119 betrifft ein zweistufiges Verfahren zur Herstellung von IPDA aus IPN, wobei ein spezieller Iminbildungsreaktor eingesetzt wird.

Zweistufig verläuft auch das in der EP-A 0 394 967 beschriebene Verfahren zur Aminierung von Carbonylnitrilen und Iminonitrilen, das auch die Herstellung von IPDA aus IPN umfaßt. Das Ausgangsprodukt wird unter Bedingungen der reduktiven Aminierung, also in Gegenwart von Wasserstoff, Ammoniak und einem Hydrierkatalysator sowie üblicherweise in Anwesenheit eines organischen Lösungsmittels bei mäßigen Temperaturen zunächst in das Aminonitril überführt; anschließend wird die Nitrilgruppe in Gegenwart eines gegenüber Nitrilgruppen hydrierwirksamen Hydrierkatalysators bei höherer Temperatur in eine Aminomethylgruppe überführt. Das Verfahren kann sowohl in Reaktoren unter Verwendung von Suspensionskatalysatoren als auch in Reaktoren unter Verwendung von Festbettkatalysatoren durchgeführt werden. Wie aus den zahlreichen Beispielen in diesem Dokument hervorgeht, wurde es als notwendig erachtet, ein strenges Temperaturprofil zu fahren, wobei die Temperatur von der ersten Stufe in 20 °C-Intervallen schrittweise auf die Temperatur in der zweiten Stufe erhöht wurde. Das zeitaufwendige Temperaturprogramm führt zu einer Erniedrigung der Raum-Zeit-Ausbeute und damit einer Minderung der Wirtschaftlichkeit des Verfahrens. Um eine ausreichende Produktqualität zu erzielen, wurden zusätzlich spezielle Promotoren eingesetzt.

Im Hinblick auf die vom cis-/trans-Isomerenverhältnis abhängigen Eigenschaften von Isophorondiamin besteht die Aufgabe der vorliegenden Erfindung darin, ein Verfahren zur Beeinflussung des cis-/trans-Isomerenverhältnisses von 3-Aminomethyl-3,5,5-trimethylcyclohexylamin bei dessen Herstellung aus 3-Cyano-3,5,5-trimethylhexanon aufzuzeigen. Die erfindungsgemäßen Maßnahmen zur Beeinflussung des Isomerenverhältnisses sollten sich in einfacher Weise in an sich bekannte Verfahren zur Herstellung von Isophorondiamin aus Isophoronnitril in Gegenwart von Ammoniak und Suspensions- oder Festbettkatalysatoren für die aminierende Hydrierung in An- oder Abwesenheit eines organischen Lösungsmittels integrieren lassen.

Gefunden wurde ein Verfahren zur Beeinflussung des cis-/trans-Isomerenverhältnisses von 3-Aminomethyl-3,5,5-trimethylcyclohexylamin (Isophorondiamin; IPDA), bei dessen Herstellung aus 3-Cyano-3,5,5-trimethylcyclohexanon (Isophoronnitril; IPN) durch aminierende Hydrierung, wobei Isophoronnitril in Gegenwart von Ammoniak und einem Suspensions- oder Festbett-Hydrierkatalysator aus der Reihe der Kobalt-, Nickel und Edelmetallkatalysatoren mit Wasserstoff bei einem Druck von 3 bis 20 MPa und einer Temperatur bis zu 150 °C umgesetzt und das erhaltene Reaktionsgemisch destillativ aufgearbeitet wird, das dadurch gekennzeichnet ist, daß man die Hydrierung in einer ersten Stufe bei 10 °C bis 90 °C und in einer sich daran anschließenden zweiten Stufe bei über 90 °C bis 150 °C, wobei der Temperaturunterschied zwischen der ersten und zweiten Stufe mindestens 30 °C beträgt, durchführt, wobei die Reaktionszeit bei Verwendung eines Suspensionskatalysators 5 bis 30 Minuten in der ersten Stufe und 30 bis 200 Minuten in der zweiten Stufe beträgt und der LHSV-Wert bei Verwendung eines Festbettkatalysators 2 bis 12 h⁻¹ in der ersten Stufe und 0,3 bis 2 h⁻¹ in der zweiten Stufe. Bevorzugt wird ein LHSV-Wert in der ersten Stufe von größer 2 h⁻¹ und in der zweiten Stufe von größer 0,8 h⁻¹.

Während durch Verwendung von beispielsweise einem Kobalt-Festbettkatalysator und Durchführung der aminierenden Hydrierung in einem Rieselbettreaktor unter Verwendung eines Ausgangsgemischs aus Isophoronnitril, Ammoniak und Methanol bei einem Druck von 60 bar und einer Temperatur von 120 °C ein cis-/trans-Isomerenverhältnis von 60 zu 40 erzielt wird, läßt sich das Isomerenverhältnis auf 80 zu 20 erhöhen, wenn die Umsetzung erfindungsgemäß bei etwa 40 °C in der ersten Stufe und 120 °C in der zweiten Stufe durchgeführt wird. Entgegen der in der EP-A 0 394 967 gegebenen Lehre ist es nicht erforderlich, die Temperatur schrittweise unter Einhaltung bestimmter Temperatursprünge und Verweilzeiten von der Temperatur der ersten Stufe auf die Temperatur der zweiten Stufe zu erhöhen. Durch die Auswahl der Temperatur der ersten und der zweiten Stufe läßt sich somit das cis-/trans-Isomerenverhältnis in einfacher Weise beeinflussen. Eine weitere Steigerung des cis-/trans-Isomerenverhältnisses gegenüber dem zuvor erwähnten ist dadurch möglich, daß die Temperatur der ersten Stufe weiter abgesenkt wird, beispielsweise auf Raumtemperatur. Durch die erfindungsgemäße Maßnahme ist es ferner möglich, kundenseitigen Wünschen nach einem ganz bestimmten Isomerenverhältnis zu entsprechen.

Die erfindungsgemäße Maßnahme zur Beeinflussung des cis/trans-Isomerenverhältnisses von Isophorondiamin läßt sich in die bekannten Verfahren zur Herstellung von Isophorondiamin integrieren, sofern diese Verfahren nicht von einem speziellen Iminbildungskatalysator Gebrauch machen. Somit lassen sich die erfindungsgemäßen Maßnahmen in Verfahren anwenden, wie sie in den einleitend gewürdigten Dokumenten offenbart wurden oder diesen Verfahren ähnlich sind.

Bekanntlich kann die aminierende Hydrierung in Anwesenheit oder Abwesenheit von Lösungsmitteln erfolgen. Vorzugsweise wird die Umsetzung in Anwesenheit von Lösungsmitteln durchgeführt, weil auf diese Weise der Druck meistens reduziert werden kann. Bevorzugte organische Lösungsmittel sind C₁- bis C₄-Alkohole, cyclische Ether, wie Tetrahydrofuran und Dioxan sowie Glykolmono- oder Glykoldimethyl- oder ethylether; besonders bewährt hat sich Methanol als Lösungmittel. Sofern ein Gemisch aus Isophoron, Ammoniak und organisches Lösungsmittel, vorzugsweise Methanol, aminierend hydriert wird, wird ein Druck im Bereich von 3 bis 8 MPa, vorzugsweise 5 bis 8 MPa, eingestellt. Die Temperatur der ersten Stufe wird in derartigen Fällen meist zwischen 20 und 80 °C und in der zweiten Stufe zwischen 100 und 140 °C liegen; die Temperaturdifferenz zwischen der ersten und der zweiten Stufe beträgt vorzugsweise mindestens 40 °C.

Bei der Suspensionshydrierung lassen sich solche Katalysatoren einsetzen, wie sie für die hydrierende Aminierung allgemein bekannt sind; insbesondere handelt es sich um Suspensionskatalysatoren mit Kobalt, Nickel oder Ruthenium als wirksames Element, wie Raney-Nickel, Raney-Kobalt und Ruthenium-Trägerkatalysatoren, etwa Ruthenium auf γ-Aluminiumoxid. Die Wirkung der Raney-Katalysatoren kann durch die Mitverwendung von Cokatalysatoren aus der Reihe von Salzen von Kobalt und Nickel gesteigert werden.

Sofern die aminierende Hydriung unter Verwendung von Festbettkatalysatoren durchgeführt wird, kann der Reaktor sowohl als Rieselbettreaktor als auch als Blasenreaktor betrieben werden. Bei der Blasenfahrweise wird das flüssige Reaktionsgemisch von unten nach oben gefördert, so daß der Reaktor stets geflutet ist; bei der Rieselbettfahrweise läßt man das flüssige Gemisch in Gegenwart von Wasserstoff über das Katalysatorbett rieseln.

Eine Rieselbettfahrweise wird bevorzugt, wobei hier insbesondere Kobalt- oder Ruthenium-Festbettkatalysatoren zum Einsatz kommen. Die erfindungsgemäß erforderlichen beiden Temperaturstufen lassen sich in einem einzigen Rieselbettreaktor mit zwei darin angeordneten Katalysatorschichten und getrennten Vorrichtungen zur Einstellung und Aufrechterhaltung der in der ersten und in der zweiten Schicht gewünschten Temperatur durchführen. Alternativ können anstelle eines einzigen Reaktors mit zwei Schichten zwei Reaktoren mit jeweils einer Schicht hintereinander geschaltet werden. Zweckmäßigerweise enthält der Rieselbettreaktor auch eine Vorrichtung zur Verteilung des auf die oberste Schicht des Reaktors aufgegebenen flüssigen Gemischs. Wasserstoff kann dem Rieselbettreaktor sowohl in einem Überschuß zugeführt werden oder entsprechend dem Bedarf zur Aufrechterhaltung des Drucks. Die Verwendung eines Wasserstoffüberschusses wird weniger bevorzugt, weil hierdurch bezüglich Ausbeute und Selektivität keine Vorteile erzielt werden und damit der apparative Aufwand vereinfacht wird, indem keine Vorrichtung zur Abtrennung des Wasserstoffüberschusses, zur Kondensation des darin enthaltenden Ammoniaks und Lösungsmittels und zur Kompression zwecks Rückführung des Wasserstoffüberschusses erforderlich sind.

Bei der Ausführung des erfindungsgemäßen Verfahrens unter Verwendung eines Rieselbettreaktors mit zwei Schichten oder unter Verwendung von zwei hintereinander geschalteten Rieselbettreaktoren, ist es zweckmäßig, auf das erste Katalysatorbett ein 10 bis 40 Gew.-% Isophoronnitril, 10 bis 40 Gew.-% Ammoniak und Methanol als Lösungsmittel enthaltendes Gemisch aufzugeben und die Hydrierung bei einem Druck von 5 bis 8 MPa und einer Temperatur von 10 bis 80 °C in der ersten Stufe und 100 bis 130 °C in der zweiten Stufe durchzuführen. Zwecks Erhalt eines hohen cis-/trans-Isomerenverhältnisses wird die Temperatur der ersten Stufe auf niedrige Werte, also insbesondere 10 bis 40 °C eingestellt.

Gemäß einer weiteren Ausführungsform des erfindungsgemäßen Verfahrens unter Verwendung eines oder zweier Rieselbettreaktoren wird für das erste Katalysatorbett ein Ruthenium-Festbettkatalysator, vorzugsweise ein Ruthenium-Trägerkatalysator mit einem Rutheniumgehalt zwischen 0,5 und 10 Gew.-% und in der zweiten Schicht ein Kobalt-Festbettkatalysator, vorzugsweise Kobalt-Trägerkatalysator mit 10 bis 70 Gew.-% Kobalt, eingesetzt. Die Schichthöhe für die erste und die zweite Schicht wird derart gewählt, daß die anspruchsgemäßen LHSV-Werte für die jeweilige Stufe resultieren. Rutheniumkatalysatoren begünstigen, wie die Anmelderin feststellte, die Bildung von cis-Isophorondiamin, beeinträchtigen aber die Ausbeute. Durch die vorerwähnte Kombination einer kleinen Schicht aus einem Rutheniumkatalysator und einer größeren Schicht aus einem Kobaltkatalysator gelingt es, insbesondere wenn die Temperatur in der ersten Stufe niedrig gehalten wird, Isophorondiamin zu erzeugen, das überwiegend in der cis-Konfiguration vorliegt.

Die besonderen Vorteile des erfindungsgemäßen Verfahrens bestehen darin, daß sich das cis-/trans-Isomerenverhältnis steuern läßt und Isophorondiamin bei den als bevorzugt dargestellten Ausführungsformen gleichzeitig in hoher Ausbeute erhalten wird. Die technischen Maßnahmen zur Steuerung des Isomerenverhältnisses sind einfach und lassen sich in bestehende Vorrichtungen integrieren. Durch die Beschränkung des Verfahrens in zwei Temperaturstufen, ohne ein langwieriges gezieltes Aufheizen, läßt sich Isophorondiamin in hoher Raum-Zeit-Ausbeute herstellen.

### Beispiel

Zwei hintereinander geschaltete Reaktionsrohre, welche mit je einer Heizvorrichtung zur getrennten Einstellung der Temperatur jedes Rohres versehen waren, wurden mit je 100 ml Hydrierkatalysator gefüllt. Die Einsatzlösung, die Isophoronnitril und Methanol enthielt und flüssiger Ammoniak wurden unmittelbar vor dem Reaktor gemischt und von oben in den ersten Reaktor gepumpt. Der Druck wurde auf 60 bar geregelt; es wurde kein H₂-Überschuß verwendet. Das flüssige Reaktionsgemisch wird in einem unter dem zweiten Reaktionsrohr angeordneten Abscheidegefäß aufgefangen.

In der Einsatzlösung waren 30 Gew.-% IPN und 70 Gew.-% Methanol enthalten. Zusätzlich wurden dem Gemisch jeweils 5,4 %, bezogen auf Isophoronnitril, eines Hochsiedergemischs aus der destillativen Aufarbeitung des Reaktionsgemischs eines vorhergehenden Ansatzes, enthaltend Reste Isophorondiamin und 3,5,5-Trimethyl-6-imino-7-aza-bicyclo-[3,2,1]-octan (Amidin) als Hauptprodukt, zugesetzt. Davon wurden 130 ml/h und zusätzlich 50 ml/h flüssiger Ammoniak gemischt und in den Reaktor gepumpt. Als Katalysator wurde ein handelsüblicher Kobaltkataysator (50 % Co auf silikatischem Träger) eingesetzt. Die Temperatur des ersten Reaktors wurde zwischen 40 und 140 °C variiert. Die Temperatur des zweiten Reaktors betrug konstant 120 °C. Die Ergebnisse sind in der Tabelle dargestellt.

| T(1. Reaktor) (°C) | Ausbeute (%) | Isomerenverhältnis cis : trans |
|---|---|---|
| 41 | 91,9 | 80 : 20 |
| 80 | 92,8 | 76 : 24 |
| 120 | 92,3 | 60 : 40 |
| 139 | 90,3 | 55 : 45 |

## Patentansprüche

1. Verfahren zur Beeinflussung des cis-/trans-Isomerenverhältnisses von 3-Aminomethyl-3,5,5-trimethylcyclohexylamin (Isophorondiamin; IPDA), bei dessen Herstellung aus 3-Cyano-3,5,5-trimethylcyclohexanon (Isophoronnitril; IPN) durch aminierende Hydrierung, wobei Isophoronnitril in Gegenwart von Ammoniak und einem Suspensions- oder Festbett-Hydrierkatalysator aus der Reihe der Kobalt-, Nickel und Edelmetallkatalysatoren mit Wasserstoff bei einem Druck von 3 bis 20 MPa und einer Temperatur bis zu 150 °C umgesetzt und das erhaltene Reaktionsgemisch destillativ aufgearbeitet wird,
dadurch gekennzeichnet,
daß man die Hydrierung in einer ersten Stufe bei 10 °C bis 90 °C und in einer sich daran anschließenden zweiten Stufe bei über 90 °C bis 150 °C, wobei der Temperaturunterschied zwischen der ersten und zweiten Stufe mindestens 30 °C beträgt, durchführt, wobei die Reaktionszeit bei Verwendung eines Suspensionskatalysators 5 bis 30 Minuten in der ersten Stufe und 30 bis 200 Minuten in der zweiten Stufe beträgt und der LHSV-Wert bei Verwendung eines Festbettkatalysators 2 bis 12 h⁻¹ in der ersten Stufe und 0,3 bis 2 h⁻¹ in der zweiten Stufe.

2. Verfahren nach Anspruch 1,
dadurch gekennzeichnet,
daß man die aminierende Hydrierung in Gegenwart eines organischen Lösungsmittels aus der Reihe der C₁- bis C₄-Alkohole, cyclischen Ether und Glykolmono- oder Glykoldi-methyl- oder -ethylether, vorzugsweise Methanol, durchführt.

3. Verfahren nach Anspruch 1 oder 2,
dadurch gekennzeichnet,
daß man in der ersten Stufe bei 20 bis 80 °C und in der zweiten Stufe bei 100 bis 140 °C hydriert.

4. Verfahren nach einem oder mehreren der Ansprüche 1 bis 3,
dadurch gekennzeichnet,
daß man die aminierende Hydrierung unter Verwendung eines oder mehrerer Festbettkatalysatoren unter Rieselbettbedingungen durchführt, wobei in einem ersten Katalysatorbett die Temperatur der ersten Stufe und in einem zweiten Katalysatorbett die Temperatur der zweiten Stufe aufrechterhalten wird.

5. Verfahren nach Anspruch 4,
dadurch gekennzeichnet,
daß man ein 10 bis 40 Gew.-% Isophoronnitril, 10 bis 40 Gew.-% Ammoniak und Methanol als Lösungsmittel enthaltendes Gemisch auf das erste Katalysatorbett gibt und das Reaktionsgemisch nach Durchrieseln durch das in einem einzigen oder in zwei Rieselbettreaktoren angeordnete erste und zweite Katalysatorbett rieseln läßt, wobei bei einem Druck von 5 bis 8 MPa und einer Temperatur von 10 bis 80 °C in der ersten Stufe und 100 bis 130 °C in der zweiten Stufe hydriert wird.

6. Verfahren nach Anspruch 4 oder 5,
dadurch gekennzeichnet,
daß man in der ersten Stufe einen Ruthenium enthaltenden Festbettkatalysator und in der zweiten Stufe einen Kobalt enthaltenden Festbettkatalysator verwendet.

## Claims

1. Process for influencing the proportion of cis-/trans isomers of 3-aminomethyl-3,5,5-trimethylcyclohexylamine (isophoronediamine; IPDA) during the preparation thereof from 3-cyano-3,5,5-trimethylcyclohexanone (isophorone nitrile, IPN) by aminating hydrogenation, wherein isophorone nitrile is reacted with hydrogen at a pressure of from 3 to 20 MPa and a temperature of up to 150°C in the presence of ammonia and of a suspension hydrogenation catalyst or fixed-bed hydrogenation catalyst selected from cobalt, nickel and precious metal catalysts and the reaction mixture obtained is worked up by distillation,
characterised in that the hydrogenation is carried out in a first step at 10°C to 90°C and in a subsequent second step at above 90°C up to 150°C, the temperature difference between the first and second step being at least 30°C, with the contact time when a suspension catalyst is used being from 5 to 30 minutes in the first step and from 30 to 200 minutes in the second step and the LHSV value when a fixed-bed catalyst is used being from 2 to 12 h⁻¹ in the first step and from 0.3 to 2 h⁻¹ in the second step.

2. Process according to claim 1,
characterised in that the aminating hydrogenation is carried out in the presence of an organic solvent selected from among the C₁ to C₄ alcohols, cyclic ethers and ethylene glycol monomethyl ether or ethylene glycol dimethyl ether or corresponding ethyl ethers and preferably methanol.

3. Process according to claim 1 or 2,
characterised in that hydrogenation is carried out in the first step at 20°C to 80°C and in the second step at 100°C to 140°C.

4. Process according to one or more of claims 1 to 3,
characterised in that the aminating hydrogenation is carried out using one or more fixed-bed catalysts under trickle-bed conditions, with the temperature of the first step being maintained in a first catalyst bed and the temperature of the second step being maintained in a second catalyst bed.

5. Process according to claim 4,
characterised in that a mixture containing from 10 to 40 wt.% of isophorone nitrile, from 10 to 40 wt.% of ammonia and methanol as solvent is applied to the first catalyst bed and the reaction mixture, after trickling through, is caused to trickle through the first and second catalyst bed arranged in a single trickle-bed reactor or in two trickle-bed reactors, hydrogenation being carried out at a pressure of from 5 to 8 MPa and at a temperature of from 10°C to 80°C in the first step and at 100°C up to 130°C in the second step.

6. Process according to claim 4 or 5,
characterised in that a fixed-bed catalyst containing ruthenium is used in the first step and a fixed-bed catalyst containing cobalt is used in the second step.

## Revendications

1. Procédé pour exercer une influence sur le rapport d'isomères cis/trans de la 3-aminométhyl-3,5,5-triméthylcyclohexylamine (Isophoronediamine ; IPDA) lors de sa production à partir de 3-cyano-3,5,5-triméthylcyclohexanone (Isophoronenitrile ; IPN) par hydrogénation aminante, dans lequel l'isophorone nitrile est mis à réagir en présence d'ammoniac et d'un catalyseur d'hydrogénation en suspension ou en lit fixe, choisi dans la série des catalyseurs au cobalt, au nickel et aux métaux nobles, avec de l'hydrogène à une pression de 3 à 20 MPa et à une température allant jusqu'à 150°C, et on traite par distillation le mélange réactionnel obtenu,
caractérisé en ce qu'
on effectue l'hydrogénation dans une première étape de 10 à 90°C et dans une deuxième étape qui lui est connectée au-dessus de 90°C à 150°C, procédé dans lequel la différence de température entre la première et la deuxième étape s'élève au moins à 30°C, dans lequel le temps de réaction lors de l'utilisation d'un catalyseur en suspension s'élève de 5 à 30 minutes dans la première étape et de 30 à 200 minutes dans la deuxième étape et dans lequel la valeur de LHSV par utilisation d'un catalyseur à lit fixe s'élève de 2 à 12 h⁻¹ dans la première étape et de 0,3 à 2 h⁻¹ dans la deuxième étape.

2. Procédé selon la revendication 1,
caractérisé en ce qu'
on effectue l'hydrogénation aminante en présence d'un solvant organique choisi dans la série des alcools en C₁ à C₄, des éthers cycliques et les éthers méthyliques ou éthyliques de glycol mono ou de glycol di-éther.

3. Procédé selon la revendication 1 ou la revendication 2,
caractérisé en ce qu'
on hydrogénise dans la première étape de 20 à 80°C et dans la deuxième étape de 100 à 140°C.

4. Procédé selon l'une ou plusieurs des revendications 1 à 3,
caractérisé en ce qu'
on effectue l'hydrogénation aminante en utilisant un ou plusieurs catalyseurs en lit fixe dans les conditions du lit à ruissellement, dans lequel dans un premier lit de catalyseur on maintient la température de la première étape et dans un deuxième lit de catalyseur on maintient la température de la deuxième étape.

5. Procédé selon la revendication 4,
caractérisé en ce qu'
on verse un mélange contenant de 10 à 40 % en poids d'isophoronenitrile, de 10 à 40 % en poids d'ammoniac et du méthanol comme solvant sur le premier lit de catalyseur et on laisse ruisseler le mélange réactionnel après la traversée par ruissellement à travers le premier et le deuxième lit de catalyseur disposé dans un réacteur unique ou dans deux réacteurs à lit à ruissellement, procédé dans lequel on hydrogénise à une pression de 5 à 8 MPa et à une température de 10 à 80°C dans la première étape et de 100 à 130°C dans la deuxième étape.

6. Procédé selon la revendication 4 ou la revendication 5,
caractérisé en ce qu'
on utilise dans la première étape un catalyseur à lit fixe contenant du ruthénium et dans la seconde étape un catalyseur à lit fixe contenant du cobalt.
